# EUROPEAN PATENT APPLICATION

(11) **EP 4 467 078 A1**
(43) Date of publication of application: **27.11.2024**
(21) Application number: 23175666.9
(22) Date of filing: 26.05.2023
(51) Int. Cl.: A61B 6/00

(54) **AUTOMATIC COLLIMATION ADAPTION FOR DYNAMIC X-RAY IMAGING**

(71) Applicant: Siemens Healthineers AG, 91301 Forchheim (DE)
(72) Inventor: Biniazan, Ramyar, 90402 Nürnberg (DE); Fieselmann, Andreas, 91052 Erlangen (DE); Fok, Wai Yan Ryana, 85748 Garching b. München (DE); Hümmer, Christian, 96215 Lichtenfels (DE); Kappler, Steffen, 91090 Effeltrich (DE)
(74) Representative: Siemens Healthineers Patent Attorneys

(57) **Abstract**

The invention relates to a method for automatically adapting a collimation (CL) for a dynamic X-ray imaging, comprising the steps of:
i) receiving criteria data (CD) for adapting the collimation (CL) from a library (LB), wherein the criteria data (CD) are assigned to a selected examination protocol and the criteria data (CD) comprise
- first criteria (C1) that are relevant for generating an adapted collimation (A-CL) based on optical image data (ID) from an examination object (0),
- second criteria (C2) that are relevant for generating a re-adapted collimation (RA-CL) based on an X-ray frame (XF) from an examination object (O),

ii) acquiring optical image data (ID) from an examination object (O) using an optical image recording device (1),
iii) generating an adapted collimation (A-CL) based on the acquired optical image data (ID) and the first criteria (C1),
iv) acquiring an X-ray frame (XF) from the examination object (O) with the adapted collimation (A-CL) using a dynamic X-ray imaging device (2),
v) performing an automatic check (EC, IC) of the adapted collimation (A-CL) of the acquired X-ray frame (XF) by checking if it meets the second criteria (C2) based on the acquired X-ray frame (XF),
vi) generating a re-adapted collimation (RA-CL) such that the second criteria (C2) are most likely fulfilled by the next acquired X-ray frame (XF) if the automatic check (EC, IC) was not passed, or maintaining the adapted collimation (A-CL) if the automatic check (EC, IC) was passed.

## Description

The invention relates to a method for automatically adapting a collimation for a dynamic X-ray imaging. In the method according to the invention, first criteria of criteria data from a library are received for adapting the collimation, wherein the first criteria are assigned to a selected examination protocol. Further, optical image data are acquired from an examination object using an optical image recording device. Based on the acquired optical image data and the first criteria, an adapted collimation is generated. Furthermore, the invention relates to a method for generating a trained AI-based model for performing an automatic check of an adapted collimation of an acquired X-ray frame. The invention also concerns an adaption device. Besides, the invention is related to a medical imaging system.

Auto collimation is a necessary part in acquiring X-ray images. Collimators are devices used to restrict and to narrow beams, which defines the border of an image for X-ray radiations. Achieving a correct collimator setting is one of the crucial aspects of improving the radiographic imaging technique. A correct collimator setting prevents unnecessary exposure of anatomy outside of the region of interest and it improves the image quality by producing less scatter radiations coming from those areas.

However, it might be possible that some necessary parts of organs that should be seen are not visible in the image. This comes from the reason that the collimator area is not wide enough to acquire all necessary parts. In other scenarios, very wide collimator areas can also happen, which cause unnecessary exposure, therefore it is against ALARA principle.

ALARA is an acronym for "As Low As Reasonably Achievable", which refers to a principle of radiation protection. When dealing with ionizing radiation, the ALARA principle requires the exposure of people, animals and material to radiation (even below the limit values) to be as low as appears feasible taking into account practical reason and weighing up the advantages and disadvantages. The word reasonably is interpreted as "as low as possible" because of the risk of cancer. The precautionary principle, which is also associated with radiation, could be counteracted in this way.

Human errors are the most common reason for failure of initial collimation and recognizing of wrong collimation.

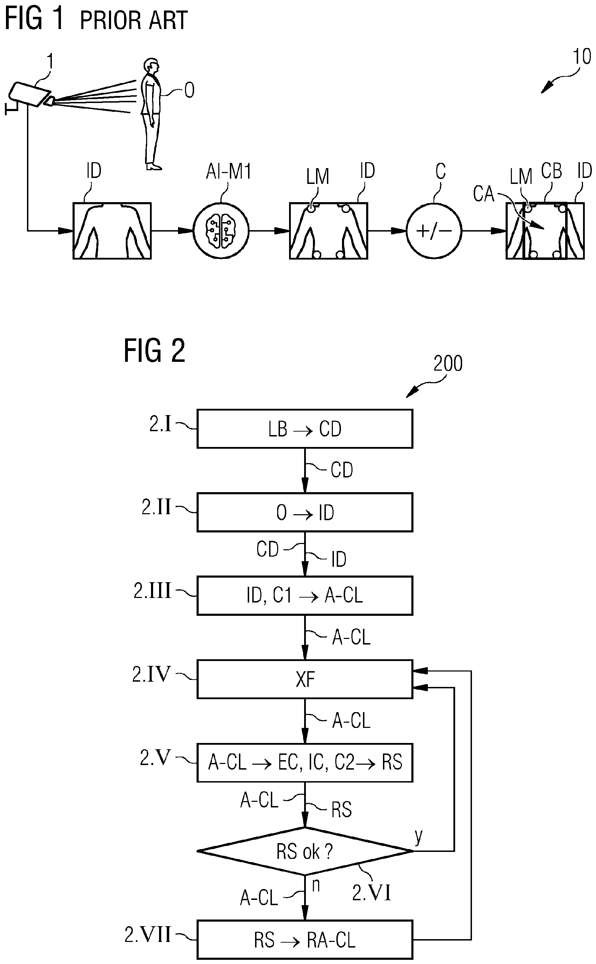

One known technique to define collimator borders is to use an RGB camera together with a depth camera. In this case, a camera in the acquisition room would first capture an image from patient and then a couple of key points would be identified on the image (by using an AI model) which later will be used to define the collimator borders. FIG 1 illustrates the steps of this technique for specific cases where the desired body part is a chest.

It must be considered that each body part will have specific key points and requirements for correct collimation, therefore, having several accurate models to detect correct key points for each body part is necessary. Furthermore, good human knowledge is also required to recognize the possible failure, where the outputs of camera models are not correct. The mentioned method has been developed and used frequently for static (radiographic) X-ray imaging where only one acquisition is required each time. However, no known technique has been developed for sequential X-ray images like those in fluoroscopy, therefore a novel approach is required to address main challenges in fluoroscopy images.

An examination method in which an X-ray camera is connected to a monitor so that the doctor can view the organ to be examined directly on the monitor is called fluoroscopy. In contrast to static X-rays, fluoroscopy is usually performed by a doctor and not by a radiologist. Small and mobile fluoroscopy devices on castors are used by the surgeons and surgical staff to visualize fractures or dislocations and for checks during the surgical setup.

Further, fluoroscopy is applied for recording representations of vessels, bile ducts and gastrointestinal sections with appropriate contrast media. Furthermore, fluoroscopy is also used for monitoring placement of probes in the body under X-ray control and for better localization of pathological processes in the body by rotating or changing the position of the patient (e.g. pulmonary nodules). Fluoroscopy is also used for observation of dynamic processes, e.g. to rule out vesicoureteral reflux, to monitor heart movement, to detect valve calcifications, to monitor swallowing movement (esophageal display) and for exclusion of leaks (fistula) after surgical interventions.

It is important to avoid mistakes in planning the region of interest and the dimensions of the collimator for minimizing X-ray load to patient.

Hence, a general problem underlying to the invention is to improve the adaption of a collimation to a dynamic medical imaging process, in particular a fluoroscopy imaging process, including sequential X-ray images.

The before-mentioned problem is solved by a method for automatically adapting a collimation for a dynamic X-ray imaging according to claim 1, by a method for generating a trained AI-based model for performing an automatic check of an adapted collimation of an acquired X-ray frame according to claim 11, by an adaption device according to claim 12 and by a medical imaging system according to claim 13.

As mentioned at the beginning, in the method for automatically, preferably AI-assisted, adapting a collimation for a dynamic X-ray imaging, preferably a fluoroscopy, criteria data are received from a library for adapting the collimation. The criteria of the criteria data are selected based on a selected examination protocol and they are related to the required information to be reflected by the X-ray images and the above-mentioned ALARA principle. A library preferably comprises a data basis including the above-mentioned criteria data.

The inventive method can be applied to any dynamic x-ray imaging methods including a continuous generation of frames of x-ray images. As an example, the inventive method can also be applied to Cone Beam CT acquired data. A "frame" comprises a single image of a film sequence. It represents the elementary unit of the film medium, analogous to the letters in writing. The frame in the static photograph is referred to as a still image.

The criteria data comprise first criteria that are relevant for generating an adapted collimation based on optical image data from an examination object and second criteria that are relevant for generating a re-adapted collimation based on an X-ray frame from an examination object. Since the X-ray frame reveals the interior of the examination object, usually, the second criteria are more detailed and precise compared to the first criteria. Preferably, the first criteria are related to the outer outlines of the examination object or the outer outlines of a portion of the examination object. The second criteria are preferably related to the interior features, in particular interior landmarks like special types of bones or organs which often enable to determine a region to be encompassed by the collimation more precisely. The examination object preferably comprises a patient, in particular a human person or an animal.

In other words, the criteria are based on keeping the radiation exposure as low as possible, but depicting all body areas relevant for a specific examination related to a selected protocol.

Further, optical image data are acquired from an examination object, preferably a human or animal patient, using an optical image recording device, preferably a camera. Furthermore, an adapted collimation is generated based on the acquired optical image data and based on the first criteria. The adaption of collimation means that the position and the dimensions of the area to be irradiated with X-rays is adapted based on the acquired optical image data. The adaption can be realized by amendment of parameter values of an adjusted collimation, in particular by amendment of the position and/or the height and/or the width of the collimation area.

For starting a dynamic X-ray imaging including a sequence of acquisitions of X-ray frames, first, an X-ray frame from the examination object is acquired with the adapted collimation using a dynamic X-ray imaging device.

In contrast to prior art, after the acquisition of the X-ray frame, an automatic check of the adapted collimation of the acquired X-ray frame is performed, wherein it is checked if the acquired X-ray frame meets the second criteria. The acquired X-ray frame comprises a 2D image.

If the automatic check was not passed, a re-adapted collimation is generated such that the second criteria of the criteria data are most likely fulfilled by the next acquired X-ray frame. If the automatic check was passed, the adapted collimation is maintained unchanged. "Most likely" means that the content of the next acquired X-ray frame is forecasted based on the previous acquired X-ray frame or sequence of acquired X-ray frames and the collimation is adjusted such that the second criteria are fulfilled by the forecasted X-ray frame. In a most simple variant, it is assumed that the content of the next acquired X-ray frame is quite similar to the last acquired X-ray frame and the collimation is adapted such that the last acquired X-ray frame, expanded or reduced by the adapted collimation, fulfills the second criteria. The information about the "expanded" last acquired X-ray frame is preferably acquired based on previously acquired X-ray frames if available or based on available anatomic or medical knowledge.

Preferably, after re-adapting or adapting the collimation, the dynamic X-ray imaging is continued by repeating the steps of starting a dynamic X-ray imaging, of performing an automatic check and of re-adapting the collimation depending on the result of the automatic check, with the re-adapted collimation as the adapted collimation if a re-adapted collimation was generated or with the adapted collimation if the automatic check was passed, until the dynamic X-ray imaging has been completed.

Advantageously, the image chain can be adapted automatically and dynamically based on the recently recorded X-ray frames. Advantageously, a feedback loop is achieved by using the acquired X-ray frames of the dynamic X-ray imaging for re-adapting the collimation of the dynamic X-ray imaging.

In the method for generating a trained AI-based model for performing an automatic check of an adapted collimation of an acquired X-ray frame, labelled input data including input data and validated result data are generated. The labelled input data comprise an X-ray frame of an examination object as input data and information related to a fulfilment of the second criteria of the criteria data as validated result data. The second criteria are relevant for generating a re-adapted collimation based on an X-ray frame from an examination object. As later described in detail, information related to a fulfilment of the second criteria of the criteria data comprises information for analysing the acquired X-ray frame regarding a fulfilment of these criteria.

A possibility to create training data based on a fluoroscopy sequence is to acquire X-ray frames from an X-ray fluoroscopy simulation based on 4D-CT-volumes, wherein "4D" represents 3D and the time as the fourth dimension.

After that, an AI-based model to be trained is applied to the labelled input data, wherein result data are generated and a training of the AI-based model is performed based on the result data generated by the AI-based model and the validated result data of the labelled input data. The training can be implemented using a cost function and a back propagation algorithm for generating an adapted AI-based model. At last, the trained AI-based model is provided to the user for applying the trained AI-based model in the method for automatically, preferably AI-assisted, adapting a collimation for a dynamic X-ray imaging according to the invention. Advantageously, an AI-based model can be adapted to an individual training database dependent on the specific type of application.

The adaption device according to the invention comprises an input interface for receiving criteria data for adapting a collimation from a library, for receiving optical image data from an examination object, and for repeatedly receiving an X-ray frame from the examination object from an X-ray imaging device.

The criteria data comprise first criteria that are relevant for generating an adapted collimation based on optical image data from an examination object and second criteria that are relevant for generating a re-adapted collimation based on an X-ray frame from an examination object.

The adaption device according to the invention also comprises an adaption unit for generating an adapted collimation based on the acquired optical image data and the criteria data.

The adaption device according to the invention further comprises an output interface for outputting the adapted collimation to the X-ray imaging device for adapting the collimation of the X-ray imaging device.

The adaption device according to the invention comprises as well a checking unit for performing an automatic check by checking if the acquired X-ray frame meets the second criteria of the criteria data.

The adaption unit of the adaption device according to the invention is also arranged for generating a re-adapted collimation based on the result of the automatic check of the checking unit such that the second criteria are most likely fulfilled by the next acquired X-ray frame if the automatic check was not passed and for keeping the adapted collimation if the automatic check was passed.

The output interface of the adaption device according to the invention is arranged for outputting the re-adapted collimation to the X-ray imaging device for re-adapting the collimation of the X-ray imaging device. The adaption device shares the advantages of the method for automatically adapting a collimation for a dynamic X-ray imaging according to the invention.

The medical imaging system according to the invention comprises an X-ray imaging device, preferably a dynamic X-ray imaging device, a library comprising criteria data for adapting a collimation of the X-ray imaging device, an optical imaging device for recording optical image data from an examination object and an adaption device according to the invention for controlling a collimation of the X-ray imaging device. A dynamic X-ray imaging device sequentially records a series of a plurality of X-ray frames. The medical imaging system shares the advantages of the adaption device according to the invention.

Some units or modules of the adaption device mentioned above, in particular the adaption unit, the checking unit and the input interface and the output interface, can be completely or partially realized as software modules running on a processor of a respective computing system, e.g. of a control device of a medical imaging system. A realization largely in the form of software modules can have the advantage that applications already installed on an existing medical imaging system can be updated, with relatively little effort, to install and run these units of the present application. The object of the invention is also achieved by a computer program product with a computer program or by a computer program that is directly loadable into the memory of a computing system, preferably of a medical imaging system. The computer program comprises program units to perform the steps of the inventive method for AI-assisted adapting a collimation for a dynamic X-ray imaging, in particular the step of receiving criteria data from a library for adapting the collimation, the step of generating an adapted collimation based on acquired optical image data and based on the first criteria of the criteria data, the step of performing an automatic check of the adapted collimation and the step of generating a re-adapted collimation. The computer program also comprises the steps of the method for generating a trained AI-based model for performing an automatic check of an adapted collimation of an acquired X-ray frame, in particular the step of generating labelled input data, the step of applying an AI-based model to be trained to the labelled input data and the step of training the AI-based model, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a harddisk or other transportable or permanently-installed carrier can serve to transport and/or to store the executable parts of the computer program product so that these can be read from a processor unit of a computing system. A processor unit can comprise one or more microprocessors or their equivalents.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the method for automatically adapting a collimation for a dynamic X-ray imaging according to the invention, the automatic check comprises an explicit check. An explicit check comprises an explicit localization and identification of image features of the acquired X-ray frame for determining if the second criteria are met based on these image features. An explicit check means that the localization and identification of image features and the determination if the second criteria are met are carried out by applying separated steps. Further, an explicit check means that the second criteria are checked based on fixed rules using human expert knowledge.

The explicit check of the adapted collimation of the acquired X-ray frame is performed by checking, based on the acquired X-ray frame, if the adapted collimation meets the second criteria of the criteria data.

Additionally to the explicit check or optionally or alternatively, an implicit check of the adapted collimation of the acquired X-ray frame is performed by applying a trained AI-based model to the acquired X-ray frame for checking if it meets the second criteria of the criteria data. An implicit check comprises the use of an end-to-end artificial neural network for determining if the second criteria are met by the acquired X-ray frame or not. An end-to-end artificial neural network means that the input data of the AI-based model is the acquired X-ray frame and the result comprises the determination if the second criteria are met or not. In other words, in addition to applying the AI-based model, there are no further steps that need to be performed for completing the implicit check. Hence, in an implicit check no rules are defined, which have to be fulfilled by the check, but the check is made based on an end-to-end trained AI-based model.

In a variant of the method for automatically adapting a collimation for a dynamic X-ray imaging according to the invention a re-adapted collimation is performed if at least one of the implicit check and explicit check was not passed. Advantageously, due to redundancy of the different types of check, a particularly robust and reliable monitoring and adaption of the collimation of a dynamic X-ray imaging is realized.

In a variant of the method for automatically adapting a collimation for a dynamic X-ray imaging according to the invention the second criteria comprise the following types of criteria:
- a predetermined anatomical structure must be visible in the optical image data or in the acquired X-ray frame or in a sequence of X-ray frames,
- the size of a possibly re-adapted collimation area should be as small as possible.

Advantageously, the anatomical structure to be examined is completely recorded by the acquired X-ray frame and the collimation area is restricted to a minimum such that an X-ray load of the patient is also restricted to a minimum.

In a further variant of the method for automatically adapting a collimation for a dynamic X-ray imaging according to the invention, the explicit check comprises the step of trying to detect landmarks related to a predetermined anatomical structure in the acquired X-ray frame for determining if a predetermined anatomical structure is visible in the acquired X-ray frame. The explicit check also comprises the step of determining the size and or position of a possibly re-adapted collimation area based on the detected landmarks and not detected landmarks. Advantageously, the size and position of the collimation area can be determined based on knowledge of anatomy and of special details of the examination to be performed by the X-ray imaging sequence.

Preferably, in the method for automatically adapting a collimation for a dynamic X-ray imaging, the step of detecting landmarks is automatically performed, preferably based on an AI-based model. Advantageously, the detection of the landmarks can be automatically performed and a human expert is not necessary for the detection task. Advantageously, the amount of training data can be reduced compared to a training of an end-to-end AI-based model as it is used for the implicit check, since the localization and identification of landmarks is assigned to an artificial neuronal network structure which is less deep than the structure of an artificial neuronal network structure assigned to an end-to-end AI-based model.

Particularly preferably, the size of a possibly re-adapted collimation area is determined such that the possibly re-adapted collimation area includes predefined landmarks. The landmarks usually mark the borders of an area to be examined in a predetermined examination. If these landmarks are visible on the acquired X-ray frame, it can be ensured that the collimation area includes the complete area for the predetermined examination.

In a variant of the method for automatically adapting a collimation for a dynamic X-ray imaging according to the invention, the implicit check comprises the determination if making the collimation area larger or smaller. Advantageously, an advice is generated which can be directly or automatically implemented for re-adapting a collimation.

Preferably, in the method for automatically adapting a collimation for a dynamic X-ray imaging according to the invention, the checks are performed for every X-ray frame or for a few X-ray frames in the beginning and followed by every other N X-ray frames, wherein N is a natural number higher than 1.

Advantageously, the number of checks can be adapted to the volatility of an imaging scenario and the computing capacity available for the checks. For example, if the rate of change of the content of the X-ray frames is low, the checks need not to be performed for every single X-ray frame. In contrast thereto, if the rate of change of the content of the X-ray frames is high, the checks must be performed with a higher frequency for ensuring the fulfilment of the ALARA principle.

In a variant of the method for automatically adapting a collimation for a dynamic X-ray imaging according to the invention, the explicit check comprises an extrapolation of the positions of the landmarks required for passing the explicit check in the following X-ray frames of the dynamic X-ray imaging and an estimation of the length and the width of the collimation area in the following X-ray frames based on the extrapolated positions. Advantageously, the collimation area can be prospectively determined for X-ray frames acquired in the future. If the X-ray frames indicate a movement of the x-ay imaging device or a movement of a region of interest to be recorded, the direction and velocity of the movement can be determined based on the recorded set of X-ray frames and an extrapolation can be determined based on the determined direction and velocity. That advantageous variant can be used for one of the following types of fluoroscopic sequences: a contrast agent flow, a walking exam, a bending exam, in particular an examination of bending a spine, an examination including the application of a barium volume, as for example a recordation of a swallowing act, of speech disorders, or of a structure swallowing test.

Preferably, the step of generating a re-adapted collimation of the method for automatically adapting a collimation for a dynamic X-ray imaging comprises making the collimation area larger if there are missed structures in the acquired X-ray frame and making the collimation area smaller if there are too many not needed structures in the acquired X-ray frame. Advantageously, the collimation area can be minimized to fulfill the ALARA-principle and at the same time the X-ray frames illustrate all the structures to be acquired for a predetermined examination.

In an also preferred variant of the method for automatically adapting a collimation for a dynamic X-ray imaging according to the invention, the step of generating a re-adapted collimation is performed using one of the following modes:
- completely automatically,
- manually by a user based on an automatically generated advice.

If using a complete automatic re-adaption of the collimation, personal resources can be saved. If the re-adaption is carried out manually, the automatically determined collimation parameter values can be checked by a human, in particular a human expert.

In a variant of the method for automatically adapting a collimation for a dynamic X-ray imaging, also the step of generating an adapted collimation based on the acquired (optical) image data and the first criteria is performed based on an AI-based model. In such a variant, it is preferred that the information of the implicit and/or explicit checks of the adapted collimation is also used for retraining that AI-based model for generating the adapted collimation. Advantageously, the initial adaption of the collimation is improved. Hence, a kind of online cross modality learning is realized, which enables to improve an initial collimation for a dynamic X-ray imaging.

In a variant of the method for generating a trained AI-based model for performing an automatic check of an adapted collimation of an acquired X-ray frame according to the invention, the labelled input data comprise one of the following data sets:
- for an explicit check:
   - an X-ray frame of an examination object as input data and detected anatomical landmarks as validated result data,
   - a fluoroscopic sequence of X-ray frames of an examination object as input data and detected anatomical landmarks as validated result data,
- for an implicit check:
   - an X-ray frame of an examination object as input data and a result of the implicit check as validated result data,
   - an X-ray frame of an examination object and/or a fluoroscopic sequence of X-ray frames of an examination object as input data and an adaption advice as validated result data,
   - an X-ray frame of an examination object and/or a fluoroscopic sequence of X-ray frames of an examination object as input data and a re-adapted collimation as validated result data.

Advantageously, specific training data are generated for training an AI-based model for performing an explicit or implicit check for a highly robust and reliable monitoring of the collimation of a dynamic X-ray imaging.

The invention is explained below with reference to the figures enclosed once again. The same components are provided with identical reference numbers in the various figures. The figures are usually not to scale.
- FIG 1: shows a schematic view on a method for adapting a collimation of a static X-ray imaging according to prior art,
- FIG 2: shows a flow chart diagram illustrating the method for automatically adapting a collimation for a dynamic X-ray imaging according to an embodiment of the invention,
- FIG 3: shows a flow chart diagram illustrating the method for generating a trained AI-based model for performing an implicit check of an adapted collimation of an acquired X-ray frame,
- FIG 4: shows a schematic diagram illustrating a medical imaging system according to an embodiment of the invention comprising an adaption device according to an embodiment of the invention.

In FIG 1, a schematic view 10 on a method for adapting a collimation of a static X-ray imaging according to prior art is depicted. In FIG 1, an optical imaging process, wherein an examination object O, in that special case a patient, is visualized using an optical image recording device 1, in that special case a combination of an RGB camera and a depth camera. One known technique to define collimator borders CB is to use an RGB camera together with a depth camera. In this case, an optical image recording device 1 in an acquisition room first captures an image ID from the patient O and then a couple of key points, i.e. landmarks LM, are identified on the image ID by using of a trained AI-based model AI-M1. FIG 1 illustrates the steps of that prior art technique for a specific case where the desired body part is a chest.

It must be considered that each body part will have specific key points, i.e. landmarks LM and requirements for correct collimation, therefore, having several accurate models to detect correct key points for each body part is necessary. Furthermore, good human knowledge is also required to recognize the possible failure where the outputs of camera models are not correct.

After detecting the landmarks LM as it is symbolized by a small picture in the middle of FIG 1, a collimation area CA with collimation borders CB is computed (symbolized by "C" for "Computing" in FIG 1) based on criteria which include that the detected landmarks LM have to be part of the collimation area CA.

The mentioned method has been developed and used frequently for static X-ray imaging where only one acquisition is required each time.

In FIG 2, a flow chart diagram 200 illustrating the method for automatically adapting a collimation for a dynamic X-ray imaging according to an embodiment of the invention is shown.

In step 2.I, criteria data CD, including first and second criteria C1, C2 for adapting the collimation are received from a library LB. The first criteria C1 are relevant for a collimation based on optical image data ID from an examination object O and the second criteria C2 are relevant for a collimation based on an X-ray frame XF from an examination object O.

In step 2.II, optical image data ID are acquired from an examination object O using an optical image recording device 1, in the embodiment illustrated in FIG 2, a camera.

In step 2.III, an adapted collimation A-CL is generated based on the acquired optical image data ID and the first criteria C1 of the criteria data CD. An "adapted collimation" means that the parameter values characterizing the dimensions of the collimation area are adapted.

In step 2.IV, an X-ray frame XF from the examination object O is acquired using the adapted collimation A-CL.

In step 2.V, an explicit check EC concerning the adapted collimation A-CL of the acquired X-ray frame XF is performed by checking if it meets the second criteria C2 of the criteria data CD.

Further, in step 2.V, also an implicit check IC concerning the adapted collimation A-CL of the acquired X-ray frame XF is performed by applying a trained AI-based model AI-M2 to the acquired X-ray frame XF.

In step 2.VI, it is determined, if the result RS of the checks EC, IC performed in step 2.V is positive or negative. The result RS is positive if both checks EC, IC were successfully passed and the result RS is negative if at least one of the checks EC, IC were not passed. In case the result RS is positive, which is symbolized with "y" in FIG 2, the method continues with step 2.IV. If the result RS is negative, which is symbolized with "n" in FIG 2, the method continues with step 2.VII.

In step 2.VII, a re-adapted collimation RA-CL is generated such that the second criteria C2 of the criteria data CD are most likely fulfilled. After that, the method continues with step 2.IV, wherein another X-ray frame XF is acquired using the re-adapted collimation RA-CL as the adapted collimation A-CL. The method continues, until the dynamic X-ray imaging is completed.

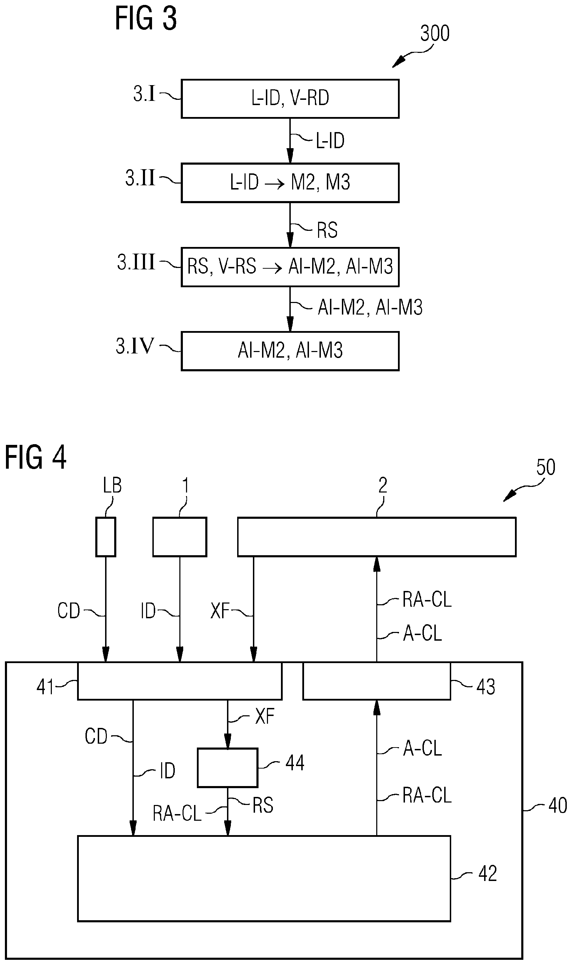

In FIG 3, a flow chart diagram 300 illustrating the method for generating a trained AI-based model AI-M2, AI-M3 for performing an implicit check IC or explicit check EC of an adapted collimation A-CL of an acquired X-ray frame XF, is depicted.

In step 3.1, labelled input data L-ID including input data IND and validated result data V-RS are generated. The labelled input data L-ID comprise one of the following data sets:
- an X-ray frame XF of an examination object O as input data and detected anatomical landmarks LM as validated result data V-RS or
- an X-ray frame XF of an examination object O as input data and a result RS of an implicit check IC as validated result data V-RS or
- an X-ray frame XF of an examination object O as input data and an adaption advice or an automatic re-adaption measurement as result RS of an implicit check IC as validated result data V-RS.

In step 3.II, an AI-based model M2, M3 to be trained is applied to the labelled input data L-ID, wherein result data RS are generated. A second model M2 is trained for performing an end-to-end determination of a re-adaption of a collimation in an implicit check IC and a third model M3 is trained for performing a localization and identification of a landmark LM in an X-ray frame XF.

In step 3.III, the AI-based model M2, M3 is trained based on the result data RS and the validated result data V-RS.

In step 3.IV, the trained AI-based model AI-M2, AI-M3 is provided for using in the method for AI-assisted adapting a collimation CL for a dynamic X-ray imaging.

In FIG 4, a schematic view on a medical imaging system 50 according to an embodiment of the invention comprising an adaption device 40 according to an embodiment of the invention is shown.

Beside the adaption device 40, the medical imaging system 50 also comprises a library LB depicted in the upper left side of the medical imaging system 50. Further, the medical imaging system 50 comprises an optical imaging device 1, which includes an RGB camera and a depth camera and which is depicted in an upper middle position in FIG 4, and an X-ray imaging device 2, depicted on the upper right side of FIG 4.

The adaption device 40, depicted in the lower part of FIG 4, comprises an input interface 41 for receiving criteria data CD from a library LB for adapting a collimation of the X-ray imaging device 2. The input interface 41 is also arranged for receiving optical image data ID of an examination object O and for repeatedly receiving an X-ray frame XF of the examination object O from an X-ray imaging device 2. The input interface 41 is illustrated in two parts in FIG 4.

Further, the adaption device 40 comprises an adaption unit 42 for generating an adapted collimation A-CL based on the acquired optical image data ID and the criteria data CD.

The adaption device 40 also comprises an output interface 43 for outputting the adapted collimation A-CL to the X-ray system 2 for adapting the collimation of the X-ray imaging device 2.

The adaption device 40 also includes a checking unit 44 for performing an explicit check EC of an acquired X-ray frame XF by checking if it meets the second criteria of the criteria data CD, or performing an implicit check IC of the acquired X-ray frame XF by applying a trained AI-based model AI-M2.

The adaption unit 42 is also arranged for generating a re-adapted collimation RA-CL based on the results RS of the explicit check EC and the implicit check IC of the checking unit 44. The re-adapted collimation RA-CL can also be generated by the checking unit 44 if an implicit end-to-end check IC is performed. As mentioned above in context with step 3.1 related to FIG 3, the result RS may also comprise an automatic re-adaption measurement.

The above-mentioned output interface 43 is also arranged for outputting the re-adapted collimation RA-CL to the X-ray imaging device 2 for re-adapting the collimation CL of the X-ray imaging device 2.

The above descriptions are merely preferred embodiments of the present disclosure but not intended to limit the present disclosure, and any modifications, equivalent replacements, improvements, etc. made within the spirit and principle of the present disclosure should be included within the scope of protection of the present disclosure.

Further, the use of the undefined article "a" or "one" does not exclude that the referred features can also be present several times. Likewise, the term "unit" or "device" does not exclude that it consists of several components, which may also be spatially distributed. Independent of the grammatical term usage, individuals with male, female or other gender identities are included within the term.

## Claims

1. Method for automatically adapting a collimation (CL) for a dynamic X-ray imaging, comprising the steps of:
i) receiving criteria data (CD) for adapting the collimation (CL) from a library (LB), wherein the criteria data (CD) are assigned to a selected examination protocol and the criteria data (CD) comprise
- first criteria (C1) that are relevant for generating an adapted collimation (A-CL) based on optical image data (ID) from an examination object (O),
- second criteria (C2) that are relevant for generating a re-adapted collimation (RA-CL) based on an X-ray frame (XF) from an examination object (O),
ii) acquiring optical image data (ID) from an examination object (O) using an optical image recording device (1),
iii) generating an adapted collimation (A-CL) based on the acquired optical image data (ID) and the first criteria (C1),
iv) acquiring an X-ray frame (XF) from the examination object (O) with the adapted collimation (A-CL) using a dynamic X-ray imaging device (2),
v) performing an automatic check (EC, IC) of the adapted collimation (A-CL) of the acquired X-ray frame (XF) by checking if it meets the second criteria (C2) based on the acquired X-ray frame (XF),
vi) generating a re-adapted collimation (RA-CL) such that the second criteria (C2) are most likely fulfilled by the next acquired X-ray frame (XF) if the automatic check (EC, IC) was not passed, or maintaining the adapted collimation (A-CL) if the automatic check (EC, IC) was passed.

2. Method according to claim 1, wherein the steps iv) to vi) are repeated with an unchanged adapted collimation (A-CL) if the automatic check (EC, IC) was passed in step (vi), or the steps iv) to vi) are repeated with the re-adapted collimation (RA-CL) as the adapted collimation (A-CL) if a re-adapted collimation (RA-CL) was generated in the last step vi), until the dynamic X-ray imaging has been completed.

3. Method according to claim 1 or 2, wherein the automatic check (EC, IC) comprises at least one or both of the following steps:
- performing an explicit check (EC) of the adapted collimation (A-CL) of the acquired X-ray frame (XF) by checking if it meets the second criteria (C2) based on the acquired X-ray frame (XF),
- performing an implicit check (IC) of the adapted collimation (A-CL) of the acquired X-ray frame (XF) by applying a trained AI-based model (AI-M2) to the acquired X-ray frame (XF) for checking if it meets the second criteria (C2).

4. Method according to any of the preceding claims, wherein the criteria data (CD) comprise the following criteria:
- a predetermined anatomical structure must be visible in the optical image data (ID) or the X-ray frame (XF),
- the size of a collimation area (CA) assigned to a possibly re-adapted collimation (RA-CL) should be as small as possible.

5. Method according to claim 3 or 4, wherein an explicit check (EC) is performed and the explicit check (EC) comprises the steps of:
- trying to detect landmarks (LM) related to a predetermined anatomical structure in the acquired X-ray frame (XF) for determining if a predetermined anatomical structure is visible in the acquired X-ray frame (XF),
- determining the size of a collimation area (CA) assigned to a possibly re-adapted collimation (CL) based on the detected and not detected landmarks (LM).

6. Method according to claim 5, wherein the step of detecting landmarks (LM) is automatically performed, preferably based on an AI-based model (AI-M3).

7. Method according to claim 5 or 6, wherein the size of the collimation area (CA) assigned to a possibly re-adapted collimation (CL) is determined such that the collimation area (CA) includes predefined landmarks (LM).

8. Method according to any of the claims 3 to 7, wherein an explicit check (EC) is performed and the explicit check (EC) comprises
- an extrapolation of the positions of required landmarks (LM) in the following X-ray frames (XF) of the dynamic X-ray imaging,
- an estimation of the length and the width of the collimation area (CA) in the following X-ray frames (XF) based on the extrapolated positions.

9. Method according to any of the claims 3 to 8, wherein an implicit check (IC) is performed and the implicit check (IC) comprises the determination if making the collimation area (CA) larger or smaller.

10. Method according to any of the claims 2 to 9, wherein the automatic check (EC, IC) is performed for:
- every X-ray frame (XF) or for
- a few X-ray frames (XF) in the beginning and followed by every other N X-ray frames (XF), wherein N is a natural number higher than 1.

11. Method for generating a trained AI-based model (AI-M2, AI-M3) for performing an automatic check (EC, IC) of an adapted collimation (A-CL) of an acquired X-ray frame (XF), comprising the steps of:
- generating labelled input data (L-ID) including input data and validated result data (V-RS), wherein the labelled input data (L-ID) comprise an X-ray frame (XF) of an examination object (O) as input data and information related to a fulfillment of second criteria (C2) of criteria data (CD) that are relevant for generating a re-adapted collimation (RA-CL) based on an X-ray frame (XF) from an examination object (O) as validated result data (V-RS),
- applying an AI-based model (M2, M3) to be trained to the labelled input data (L-ID), wherein result data (RS) are generated,
- training the AI-based model (M2, M3) based on the result data (RS) and the validated result data (V-RS),
- providing the trained AI-based model (AI-M2, AI-M3).

12. Adaption device (40), comprising:
- an input interface (41)
- for receiving criteria data (CD) for adapting a collimation (CL) from a library (LB), wherein the criteria data (CD) are assigned to a selected examination protocol, wherein the criteria data (CD) comprise:
- first criteria (C1) that are relevant for generating an adapted collimation (A-CL) based on optical image data (ID) from an examination object (O),
- second criteria (C2) that are relevant for generating a re-adapted collimation (RA-CL) based on an X-ray frame (XF) from an examination object (O),
- for receiving optical image data (ID) from an examination object (O), and
- for repeatedly receiving an X-ray frame (XF) from the examination object (O) from an X-ray imaging device (2),
- an adaption unit (42) for generating an adapted collimation (A-CL) based on the acquired optical image data (ID) and the first criteria (C1),
- an output interface (43) for outputting the adapted collimation (A-CL) to the X-ray imaging device (2) for adapting the collimation (CL) of the X-ray imaging device (2),
- a checking unit (44) for performing an automatic check (EC, IC) of the acquired X-ray frame (XF) by checking if the acquired X-ray frame (XF) meets the second criteria (C2) of the criteria data (CD),
wherein
- the adaption unit (42) is also arranged for generating a re-adapted collimation (RA-CL) such that the second criteria (C2) are most likely fulfilled if the automatic check (EC, IC) was not passed and or for maintaining the adapted collimation (A-CL) if the automatic check (EC, IC) was passed,
- the output interface (43) is arranged for outputting the re-adapted collimation (RA-CL) to the X-ray imaging system (2) for re-adapting the collimation (CL) of the X-ray imaging device (2) or the maintained adapted collimation (A-CL) .

13. Medical imaging system (50), comprising:
- an X-ray imaging device (2),
- a library (LB) comprising criteria data (CD) for adapting a collimation (CL) of the X-ray imaging device (2),
- an optical imaging device (1) for recording optical image data (ID) from an examination object (O),
- an adaption device (40) according to claim 12 for controlling a collimation (CL) of the X-ray imaging device (2).

14. A Computer program product comprising instruction which, when the program is executed by a computer, cause the computer to carry out the steps i) to vi) of the method for automatically adapting a collimation (CL) for a dynamic X-ray imaging according to any of claims 1 to 10 or the steps of generating labelled input data (L-ID), of applying an AI-based model (M2, M3) to be trained to the labelled input data (LID) and of training the AI-based model (M2, M3) of the method for generating a trained AI-based model (AI-M2, AI-M3) for performing an automatic check (EC, IC) of an adapted collimation (A-CL) of an acquired X-ray frame (XF) according to claim 11.

15. A computer program comprising instructions which, when executed by a computer, cause the computer to carry out the steps i) to vi) of the method for automatically adapting a collimation (CL) for a dynamic X-ray imaging according to any of claims 1 to 10 or the steps of generating labelled input data (L-ID), of applying an AI-based model (M2, M3) to be trained to the labelled input data (L-ID) and of training the AI-based model (M2, M3) of the method for generating a trained AI-based model (AI-M2, AI-M3) for performing an automatic check (EC, IC) of an adapted collimation (A-CL) of an acquired X-ray frame (XF) according to claim 11.
